# EUROPEAN PATENT SPECIFICATION

(11) **EP 0 513 228 B1**
(45) Date of publication and mention of the grant of the patent: **29.09.1999**
(21) Application number: 91904845.4
(22) Date of filing: 07.02.1991
(51) Int. Cl.: A61B 17/16

(54) **CURVED BORE DRILLING APPARATUS**
VORRICHTUNG ZUM BOHREN EINER GEKRÜMMTEN BOHRUNG
APPAREIL DE PERCAGE D'ALESAGES COURBES

(30) Priority: 09.02.1990 WO PCT/US90/00771; 18.04.1990 US 510743
(43) Date of publication of application: 19.11.1992
(73) Proprietor: Romano, Jack W., Seattle, WA 98155 (US)
(72) Inventor: Romano, Jack W., Seattle, WA 98155 (US)
(74) Representative: Spall, Christopher John
(86) International application number: US9100839
(87) International publication number: WO9111962

(56) References cited:
- WO-A-87/03522
- DE-C- 133 162
- GB-A- 180 030
- US-A- 1 223 938
- US-A- 1 698 952
- US-A- 1 822 330
- US-A- 2 291 413
- US-A- 2 666 430
- US-A- 2 747 384
- US-A- 2 905 178
- US-A- 2 960 892
- US-A- 3 554 192
- US-A- 3 628 522
- US-A- 3 697 188
- US-A- 3 815 605
- US-A- 4 257 411
- US-A- 4 265 231
- US-A- 4 312 337
- US-A- 4 345 601
- US-A- 4 421 112
- US-A- 4 541 423
- US-A- 4 590 929
- US-A- 4 622 960
- US-A- 4 941 466

## Description

### Field of the Invention

This invention relates to apparatus and methods for forming or drilling one or more curved bores or holes into a solid material; and, more particularly, to drilling apparatus having a remotely actuated, pivotal rocker arm and curved guide means which guide and advance a cutting means through a predetermined curved path within a solid material.

### Background of the Invention

Many industries, including the health profession, often desire to produce curved bores. Cannulas, lumens, ducts, and other types of tubular and curved conduits are each needed for particular purposes. Machinery often needs such conduits to transport fuel, coolants, cutting fluids, gases, products, and/or by-products. Similarly, curved conduits are often needed within the plumbing, electrical, and heating industries to transport fluid, wiring, and heating around restricted comers.

Heretofore, machining for curved conduits has been near impossible. Curved bores within solid objects have been particularly difficult. A plurality of straight bores may be used at various angles in an attempt to round difficult corners. Other options are to form a precast mold having a removable curved plug for a core, or to prepare multiple piece vacuum or injection molds. Such tooling techniques are time-consuming and expensive.

The need for curved bores also exists within the field of orthopedic surgery. For example, it is often desirable to secure a suture or a wire to a bone to: secure a tissue, such as ligament; immobilize adjacent bone structures; and/or reduce a fracture. Traditional practice is to make two independent straight bores at intersecting angles at some angle less than 180' within the bone structure. A curved needle is then gradually forced through the bone from one bore hole to the other. This practice is highly susceptible to operational error. Such a procedure is also very time consuming if the bore holes do not intersect or if the permitted working area is restricted.

In some instances severe damage can be caused to the surrounding bone structure and soil tissue. By its very nature, this procedure removes more bone than is desirable. It is also not uncommon to break a needle within hard bone structure by using too much force in attempting to force a curved needle through two straight holes, thus, necessitating additional time and inconvenience to retrieve the broken needle fragments. This greatly increases the expense to perform the operation. Additional anesthesia is required which also increases the risk associated with the operation.

The following surgical devices were created in an attempt to overcome such difficulties.

Scheller, Jr., et al. (United States Letters Patent No. 4,265,231; issued May 5, 1981) lists a number of specific examples of such operations wherein a curved bore is advantageous and describes one known method and apparatus for forming curved bores. The Scheller device is an example of the use of a flexible drill containing cannula of a predetermined curvature which may be hand-manipulated through its curved path within bone.

As may be appreciated, the manipulation of such a cannula requires a considerable amount of space and it is thus of limited utility where access is limited. The space requirement for operation of the instrument also necessitates a considerably larger skin incision. Not only does the manipulation of the hand held cannula and drive motor require additional space, but the cumbersome size and shape of the cannula itself severely limits the utility of the Scheller device.

Other examples of the use of a rigid curved cannula are illustrated in Barber (U.S. Letters Patent No. 2,541,423; issued September 17, 1985) and in Donohue (U.S. Letters Patent No. 4,312,337; issued January 26, 1982). These devices suffer from the same limitations discussed above with respect to the Scheller method and apparatus.

In each of the cited patents, the cannula is used to push, pull or advance the drill shaft through a curved path determined by the operator's manipulation of the entire instrument. Allowance must be made for the shape of the preformed cannula.

The primary problems with the apparatus and methods of the prior art are: the time consuming nature of the procedures; the inability to operate in a restricted area; and the possibility of severe damage to bone and/or to surrounding tissue. The results of these drawbacks may have far reaching effect in terms of the cost for surgery, the degree of scarring, and the recovery and rehabilitation time required, as well as increased anesthesia risks.

Furthermore, modern day trends of reducing incision size and decreasing tissue violation do not lend themselves well to the sometimes crude and inexact methods currently in practice, or described in any of the cited patents. For example, in the practice of arthroscopic surgery an instrument is inserted into a joint cavity through an extremely small puncture incision which is only large enough to allow entrance of the instrument. The cavity and manipulation of the instrument is observe by means of a scope device inserted through a second near size incision. Curved bone drilling under these conditions is presently non-existent, and would be extremely difficult if not impossible with known bone drilling devices.

The inventor believes that the listed patents and known prior art taken alone or in combination neither anticipate nor render obvious the present invention. These citations do not constitute an admission that such disclosures are relevant or material to the present claims. Rather, these citations relate only to the general field of the disclosure and are cited as constituting the closest art of which the inventor is aware.

### Summary of the Invention

The present invention may be used within a wide variety of industries wherein simple, easy, inexpensive apparatus and methods are desired to form one or more curved bores within a solid material

According to one aspect of our invention, we provide an apparatus for drilling a curved bore within a material comprising:
(a) a support structure;
(b) a rocker arm pivotally secured to said support structure at a pivot point, said pivot point defining an axis of rotation, said rocker arm defining a rotating link or crank which rotates or oscillates about said pivot point, said rocker arm having a first end located near said pivot point and having a second end extending radially outward from said pivot point;
(c) curved guide means having a first end rigidly attached to or integral with said second end of said rocker arm, said curved guide means having an extended second end which cantilevers outwardly from said rocker arm, said curved guide means generally laying within an arc defined by a rotational path of said second end of said rocker arm as said rocker arm is rotated about said pivot point, said pivot point being located near a protrusion or edge of said support structure;
(d) means for cutting said material within confines of said curved bore;
(e) means for securing said cutting means to said second end of said curved guide means;
(f) means for remotely actuating rotation of said rocker arm, and
the cutting means comprising a cutting head and a flexible drilling energy carrier for connection to a remote power source said cutting head being adapted to advance and retract through a predetermined path first outward away from the support structure then at least partially inward toward the support structure, and characterised by the curved guide means having an outwardly extending receiving channel into which said flexible drilling energy carrier of said cutting means is guided, received and retained, said channel receiving, retaining and releasing said flexible drilling energy carrier as said cutting means and said curved guide means is advanced and retracted to drill a curved bore through said material, and in that said actuation means comprises a connecting rod or push/pull flexible linkage pivotally secured to either said rocker arm or to said curved guide means, movement of said connecting rod or linkage causing said rocker arm, said curved guide means, and said cutting head to advance or retract through said predetermined path.

According to yet another aspect of our invention, we provide a method for drilling a curved bore within a material, which method comprises: moving an elongate flexible drilling energy carrier along a first direction or approach path toward and away from a curved guide means, said guide means being carried by a rocker means generally lying within an arc defined by a rotational path centered about an axis of rotation; and remotely actuating rotation of the curved guide means by translating a rigid push rod connected to the curved guide means along an arcuate path of an extent of more than 90°, and diverting the flexible drilling energy carrier by engagement with the curved guide means to pass along a curvilinear path having a curvature of more than 90° defined by the rotational path centered about the axis of rotation, while supplying drilling energy along the elongated flexible drilling energy carrier to a cutting head secured to said curved guide means and disposed at an end of the drilling energy carrier, and thereby advancing the cutting head toward the material to bore a hole along the predetermined curved path within the material and retracting the cutting head to an initial position thereby withdrawing the curved guide means and the cutting head from within the material; but excluding any method within the meaning of Article 52(4) EPC.

The claimed apparatus and methods further contemplate use of traditional and/or non-traditional machining processes, including mechanical, electrochemical, and thermal processes. Such cutting means may be remotely actuated by a wide variety of actuating means. Furthermore, the present invention allows the cutting means to have a wide range of orientations with respect to the bored surface.

The present invention is a compact, functional, efficient, reliable, reusable, durable, rugged, easily constructed, inexpensive and economical to manufacture apparatus that requires minimal manipulation and is simple to use. The present invention not only increases the speed and simplifies the procedure to form curved bores, it also provides an inexpensive, unobtrusive drilling apparatus which requires less access room for operation and does not damage adjacent material. During use, it is unnecessary to alter the angle of approach of a support structure, such as the housing of the apparatus, with respect to the material to be drilled. This enables curved bores to be formed within material, such as bone, through a very small incision which allows access to a deep bone structure, without damaging adjacent bone structure or tissue. The present invention also overcomes all of the previously mentioned disadvantages.

The support structure may take any form which appropriately orients the apparatus with respect to the material to be drilled. The support structure may comprise an independent, dedicated support stand, an arm extending from a larger machine or structure, a hand-held housing unit, or similar structure which meets the particular needs of the user.

The rocker arm is pivotally secured to the support structure at a fixed pivot point and rotates about an axis of rotation. This pivot point is extremely important to the present invention. In essence, the movement of the guiding means and cutting means, which will be described further below, is dependent upon their rotation about this fixed pivot point. Likewise, the bore to be formed within the material will also depend upon a predetermined curved path defined by a rotation about this fixed pivot point.

Once a fixed spacial relationship is achieved between the drilling apparatus and the material to be drilled, rotation of the rocker arm, curved guide means, and cutting means permits a curved bore to be formed within the material.

The pivot point may comprise a cylindrical pivot pin which pivotally secures the rocker arm to the support structure. The rocker arm defines a rotating link or crank which rotates and/or oscillates about the fixed pivot point. The rocker arm has a first end located near the fixed pivot point and has a second end extending radially outward from the fixed pivot point.

The curved guide means defines an arcuate channel having a first end rigidly attached to the second end of the rocker arm. The curved guide means has a extended second end which cantilevers outwardly from the rocker arm. The curved guide means generally lies within an arc define by the rotational path of the second end of the rocker arm as the rocker arm is rotated about its fixed pivot point. The length of the rocker arm and the length and cross-sectional area of the curved guide means are dependent upon the desired curve, length, and cross-sectional area of the curved bore to be formed.

The pivot point is located near a protrusion or edge of the support structure in such a manner that when the rocker arm is pivoted or rotated, the second end of the curved guide means extends away from an opening of the support structure passing through a predetermined curved path. Thus, if a material is juxtaposed near the pivot point and support structure, rotation or pivotal movement of the rocker arm will urge the second end of the curved guide means toward the material.

The forward, cantilevered, or extended second end of the curved guide means is provided with means for securing the cutting means thereto. Such securing means may comprise a collar, bearing, a bushing, or other structure to hold and retain the cutting means in place.

The cutting means may comprise any apparatus or method which is capable of cutting a curved bore. Traditional or nontraditional machining processes may be used, including mechanical, electrochemical, and thermal processes. In one embodiment, the inventor prefers to use a electric discharge machining (EDM) process.

An appropriately powered pivotal connection can be made at the fixed pivot point of the rocker arm to transfer power from a remote power source to a leading, boring tip or cutting head secured near the second end of the curved guide means. Electric discharge machining (EDM) allows for such a connection.

In another embodiment, a rotary cutting means is used. The cutting means requires the use of an elongated flexible drilling energy carrier which, with a rotary cutter, is a flexible shaft. The drilling energy carrier is directly connected to a remote power source. To accommodate such a connection, the curved guide means is provided with an outwardly extending receiving channel which is used to guide, receive, and retain the flexible drilling energy carrier of the cutting means. For example, some processes require rotational power obtained from a remote rotational power source to be transmitted through a flexible drill shaft to rotate a leading cutting head. To use such a cutting means, the second end of the curved guide means is provided with a securing means which permits rotation of the secured cutting head. The curved guide means is also provided with an exterior channel which is designed to guide, receive and retain the rotatable, flexible shaft. As the rocker arm is pivoted, the curved guide means and cutting head are advanced toward the material. The channel of the curved guide means receives and retains the flexible shaft as the cutting head and curved guide means advance toward and through the material.

Thus, when the rocker arm is pivoted, the curved guide means guides and advances the cutting means away from the support structure to bore a hole along a predetermined curved path within the material.

After the bore has been created, the motion of the rocker arm is reversed and the curved guide means and cutting means are removed from within the material. The insertion and retraction of the curved guide means and cutting means into and from the material is defined by the oscillation of the rocker arm.

The pivotal rocker arm, curved guide means, and cutting means may be remotely actuated by a wide variety of actuation means. The actuation means urges the rocker arm to rotate, which in turn urges the curved guide means and cutting means to advance or retract through the predetermined curved path.

A connecting rod or push/pull linkage is pivotally secured to either the rocker arm or to the curved guide means. Preferably, the connecting rod is secured at a juncture or intersection of the rocker arm and of the curved guide means. Appropriate movement of the connecting rod will cause the rocker arm, curved guide means, and cutting means to advance or retract through the predetermined curved path.

In a first embodiment of the present invention the actuation means comprises a double parallel-crank mechanism. The rocker arm, and two different crank systems are rotationally secured to the support structure. A single connecting rod is then connected to the rocker arm and to each of the two crank systems. A first end of the connecting rod is pivotally secured to the rocker arm. The first crank system is pivotally connected to the connecting rod at an intermediate location along the length of the connecting rod. A second end of the connecting rod is pivotally connected to the second crank systems. In essence, the support structure, rocker arm, connecting rod, first crank system and second crank system form a plane parallelogram. Means for rotating the first and second crank systems are also provided. For example, one or more tension cords, chains, cables or the like may be used to rotate the first and/or second crank systems. Similarly, a spring may be used to urge one or more crank systems toward rotation or to retract the crank systems from an earlier rotation. The tension cords may then be connected to an easily controlled lever, trigger, switch, or the like.

The first and/or second crank systems may one or more pulleys incorporated therein. Such pulleys share the same axis of rotation as their corresponding crank system. The above mentioned tension cord may be passed around one or more of the pulleys to provide smooth, easy actuation and rotation of the plane parallelogram and attached curved guide means and cutting head.

In a second embodiment the pulleys and tension cords are replaced with a rack and pinion system.

In a further embodiment of the actuation means a crossed-crank mechanism may be used.

In still further embodiments, multiple rocker arms, curved guide means, cutting means, and actuation means may be used in unison or within a single apparatus.

The present invention allows the cutting means to have a wide range of orientations with respect to the bored surface. This is accomplished by controlling the path and angle of rotation of the curved guide means and cutting means, and by placing the curved guide means and cutting means at various angles with respect to the remaining elements of the apparatus and/or with respect to the bored material.

Due to pressure to reduce medical costs, greater emphasis is made to increase efficiency and reduce the time required to perform various procedures. Any reduction in time, and increase in accuracy and efficiency is therefore of great significance. Further, the modem trend is toward procedures which reduce incision size and tissue violation. The latter trend is demanded not only for cost reduction and shortened recovery time, but also to reduce pain, scarring, rehabilitation time, anesthesia risk, required pain medication, and wage loss during recovery.

The present invention provides an apparatus and methods to produce a curved bore with great accuracy and with minimum damage to adjacent bone and tissue within an extremely limited incision. For example, curved bores may now be formed in shoulder joints at difficult locations using safe, conventional puncture sites. When used, this invention allows the attachment of filament to bone in a exact and efficient manner, saving time and cost, and permitting the operation to be performed within very small, deep incision, heretofore impossible with prior art devices. As a consequence of its efficiency, the present invention also decreases post-operative therapy and expense.

These and other objects and advantages of the present invention will become more readily apparent upon reading the following disclosure and referring to the attached drawings.

### Brief Description of the Drawings

The foregoing aspects and many of the attendant advantages of this invention will become more readily appreciated as the same becomes better understood by reference to the following detailed description, when taken in conjunction with the accompanying drawings, wherein:
FIGURE 1 is a side-elevational view of a first embodiment of the drilling apparatus as taught herein with a portion of a left wall of a support structure housing removed to illustrate an interior rocker arm, curved guide means, cutting means, and actuation means.
FIGURE 2 is a plan view of the first embodiment shown in FIGURE 1, wherein a portion of an upper wall of the housing is removed to illustrate the interrelationship between the interior rocker arm, curved guide means, cutting means, and actuation means.
FIGURE 3 is a front-elevational view of the first embodiment shown in FIGURES 1 and 2 with a portion of the front and upper walls of the housing removed.
FIGURE 4 is a partially sectioned, side-elevational view of a second embodiment of the invention, wherein the actuation means comprises a rack and pinion system.
FIGURE 5 is a partially sectioned, plan view of the second embodiment shown in FIGURE 4 with a portion of the upper wall of the housing removed to better show the rack and pinion system.
FIGURE 6 is a side-elevational view of a third embodiment of the invention with a portion of the left wall of the housing removed.
FIGURE 7 is a partial side-elevational view of the support structure, rocker arm, curved guide means having an external receiving channel, and cutting means having a flexible optical conduit which is directly connected to a cutting head.
FIGURE 8 is a schematic, partially sectioned, side-elevational view of a fourth embodiment of the present invention which incorporates the use of an electric discharge machining (EDM) process.
FIGURE 9 is a schematic, plan view of the fourth embodiment shown in FIGURE 8.
FIGURE 10 is a partially sectioned, side-elevational view of a fifth embodiment of the present invention, wherein the actuation means comprises a crossed-crank mechanism.
FIGURE 11 is a partially sectioned, side-elevational view of a fifth embodiment shown in FIGURE 10, wherein the rocker arm and curved guide means are partially rotated to extend the cutting means past the support structure along a predetermined curved path.
FIGURE 12 is a partially sectioned, plan view of the fifth embodiment as shown in FIGURE 10.
FIGURE 13 is a partial, isometric view of an alternative drilling apparatus as seen in FIGURE 14 and FIGURE 15.
FIGURE 16 is a partial, cross-sectional, side-elevational view of the sixth embodiment shown in FIGURES 13 and 15, and wherein the rocker arm, curved guide means, and cutting head pivot within a plane that is different from that of the rigid drive shaft.

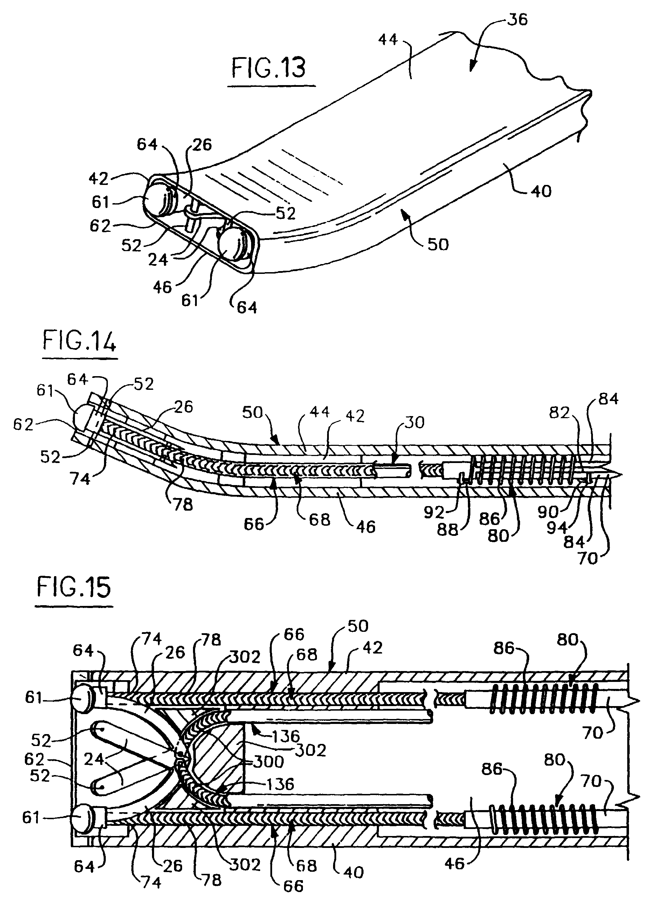

FIGURE 15 is a partial, cross-sectional, plan view of the sixth embodiment shown in FIGURES 13 and 14 with an upper wall of the housing removed to illustrate use of multiple rocker arms, curved guide means, cutting means, and actuation means within a single apparatus.

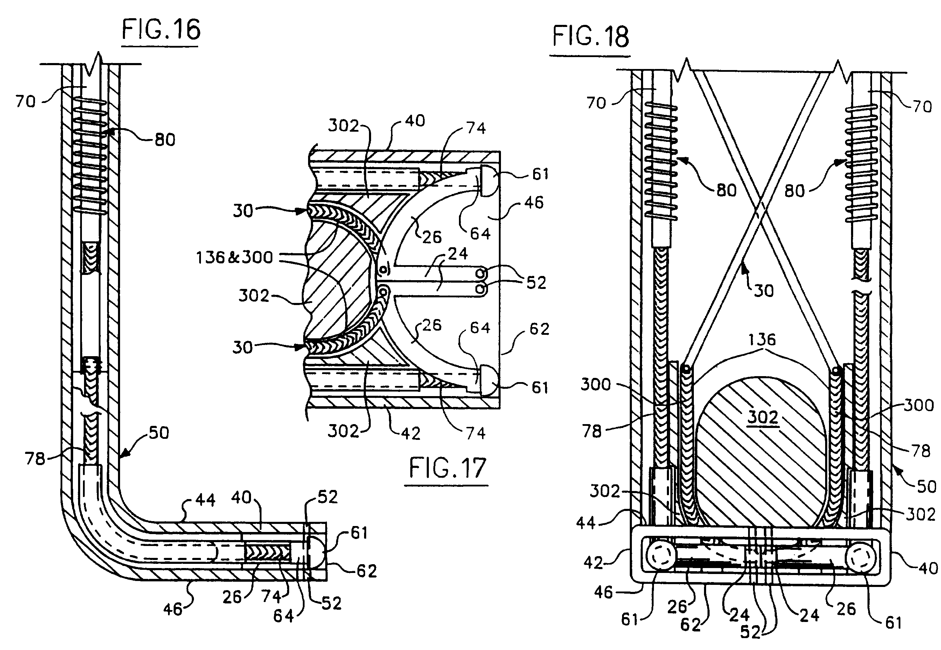

FIGURE 16 is a partial, cross-sectional, side-elevational view of a yet further alternative apparatus shown in FIGURES 17 and 18, wherein the rigid drive shafts rest within a plane which is generally perpendicular to the plane within which the multiple rocker arms, curved guide means, and cutting means rotate.
FIGURE 17 is a partial, plan view of the alternative shown in FIGURES 16 and 18.
FIGURE 16 is a partial, cross-sectional, front-elevational view of the alternative shown in FIGURES 16 and 17.

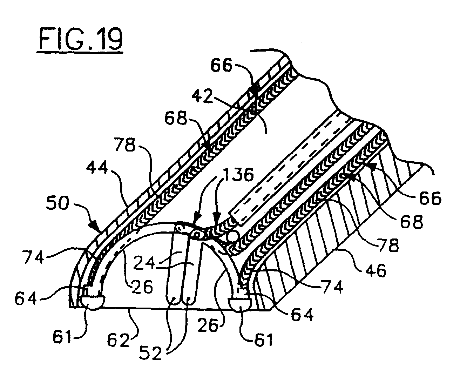

FIGURE 19 is a partial, cross-sectional, side-elevational view of a still further alternative apparatus.

It will be understood that the embodiment of Figures 13-19 do not fall within the scope of the invention in so far as the energy carrier means does not pass through an angle greater than ninety degrees during cutting of the bore.

One should understand that the drawings are not necessarily to scale and the elements are sometimes illustrated by graphic symbols, phantom lines, diagrammatic representations, and fragmentary views. In certain instances, the inventor may have omitted details which are not necessary for an understanding of the present invention or which render other details difficult to perceive.

### Detailed Description of the Preferred Embodiment

### Best Mode for Carrying Out the Invention

Referring to the drawings, wherein like numerals indicate like parts, a curved bore drilling apparatus 20 of the present invention comprises: a support structure 22; a rocker arm 24; curved guide means 26; cutting means 28; and actuation means 30.

### Support Structure

Support structure 22 may take any form which appropriately orients drilling apparatus 20 with respect to a material to be drilled. For example, support structure 22 may comprise an extension or arm of a separate larger machine or structure (not shown).

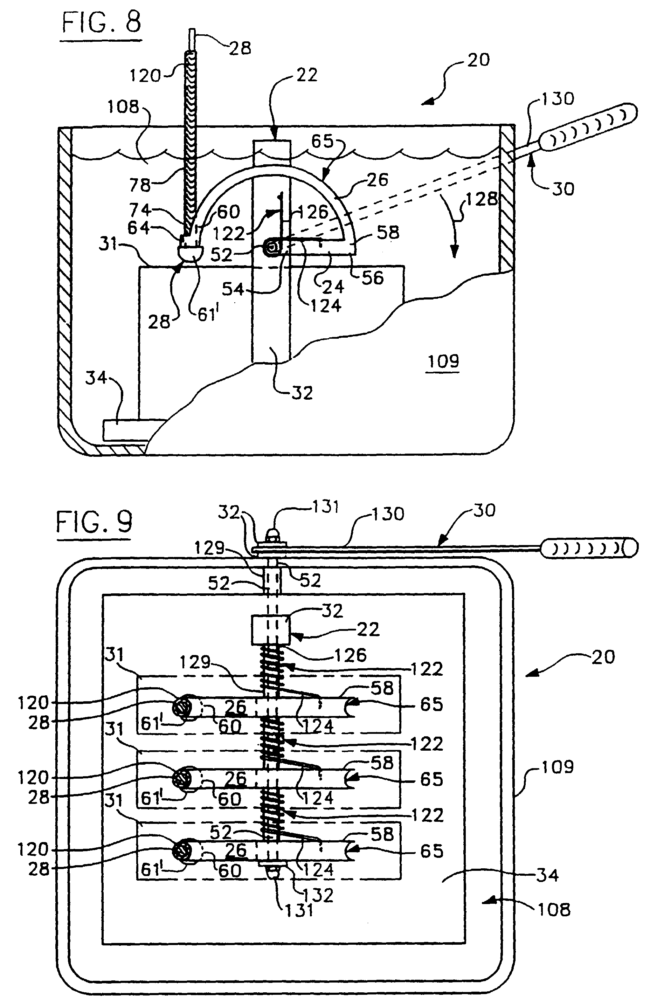

Support structure 22 may also be an independent support stand specifically dedicated to supporting the remaining elements of drilling apparatus 20. As shown in FIGURES 8 and 9, support structure 22 may simply comprise a upright post 32 which is secured to a horizontal base 34. Upright post 32 or horizontal base 34 may serve other purposes, such as holding the material 31 to be drilled.

In the preferred embodiment, support structure 22 comprises a housing 36 which defines the parameters of an enclosure located therein. Housing 36 supports, protects, and contains rocker arm 24, curved guide means 26, cutting means 28, and actuation means 28. Housing 36 has an opening into enclosure 38. The opening is located near rocker arm 24, curved guide means 26, and cutting means 28.

For easy use, housing 36 may be a hand-held unit of any size and shape which meets the particular needs of a user.

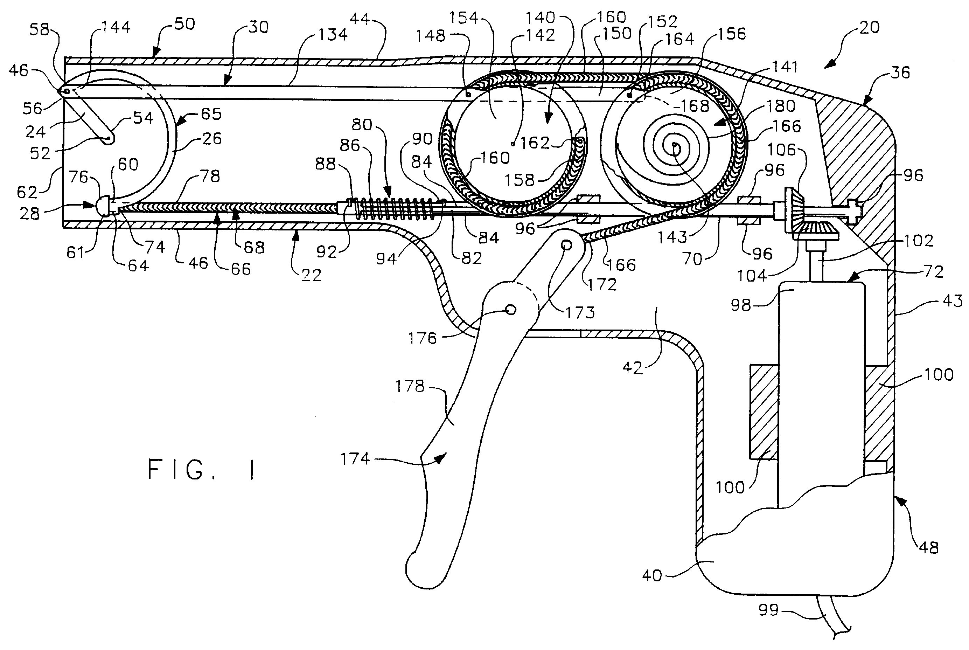

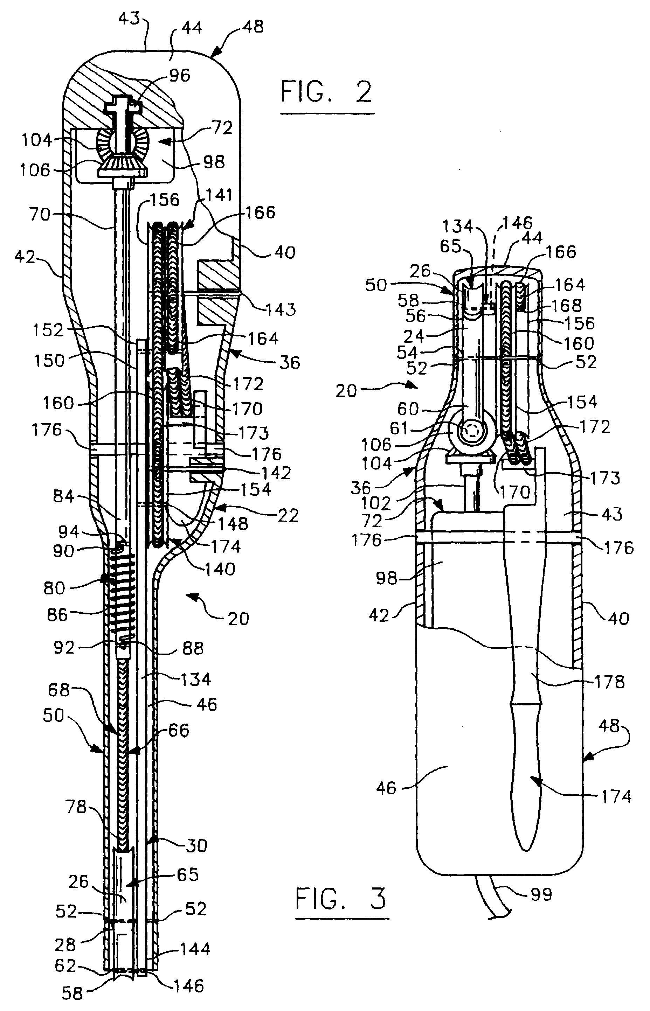

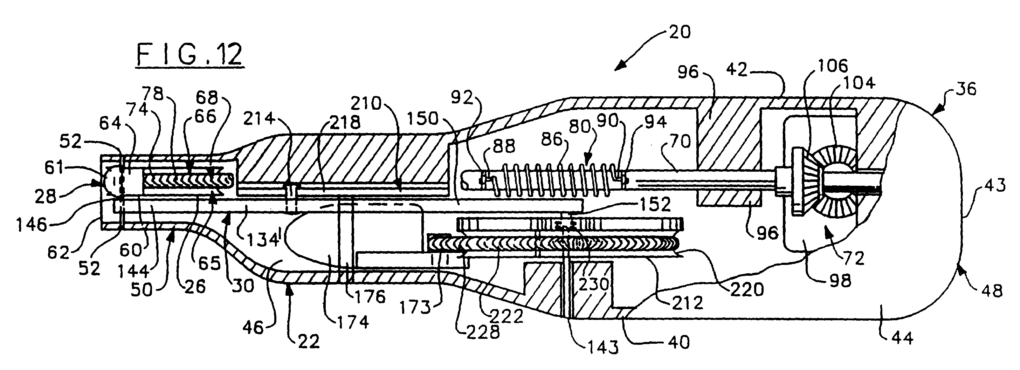

For use in arthroscopic surgery, housing 36 has a pistol shape which is easily held. As seen in FIGURE 1, housing 36 may comprise: a left wall 40, a right wall 42, a rear wall 43, an upper wall 44, and a lower wail 46, which generally define a handle portion 48 and a neck portion 50 of housing 36. Typically, neck portion 50 of housing 36 is extremely thin and narrow to allow placement of rocker arm 24, curved guide means 26, and cutting means 28 deep within a small, narrow incision (not shown). However, to better illustrate the interrelationship between housing 36, neck portion 40, rocker arm 24, curved guide means 26, cutting means 28, and actuation means 30, the size and dimensions of such elements have been substantially enlarged within FIGURES 1-6 and 11-20.

### Rocker Arm

In the preferred embodiment of the present invention, the curved guide means is mounted for rotation about a fixed axis relative to the support structure. A rocker arm 24 is pivotally secured to housing 36 at a fixed pivot point which defines its axis of rotation. As shown in FIGURE 1, the fixed pivot point may comprise a pivot pin 52 which pivotally secures rocker arm 24 to housing 36. Pivot pin 52 may be secured to either left wall 40 or to right wall 42. In the preferred embodiment, pivot pin 52 is secured to both left wall 40 and to right wall 42 and spans the distance within enclosure 38 between such walls.

Rocker arm 24 defines a rotating link or crank which rotates and/or oscillates about pivot pin 52. Rocker arm 24 has a first end 54 located near pivot pin 52 and has a second end 56 which extends radially outward from pivot pin 52.

### Curved Guide Means

Curved guide means 26 defines an arcuate guide channel having a first end 58 which is rigidly attached to or integrated with second end 56 of rocker arm 24. Rocker arm 24 and curved guide means 26 may be attached together by any appropriate means such as by adhesion, bolting, welding, or the like. In the preferred embodiment, rocker arm 24 and curved guide means 26 are formed from a single piece of material. Such single piece of material is then appropriately bent to form second end 56 of rocker arm 24 and first end 58 of curved guide means 26.

Curved guide means 26 also has an extended second end 60 which cantilevers outwardly from second end 56 of rocker arm 24. Curved guide means 26 generally lies within an arc that is defined by the rotational path of second end 56 of rocker arm 24 as rocker arm 24 is pivoted and/or rotated about pivot pin 52.

The angle of curvature, cross-sectional area, and depth of the curved bore to be formed, are dependent upon various factors including: the longitudinal length of rocker arm 24; the curved length of curved guide means 26; the cross-sectional area of curved guide means 26; the angle of rotation of rocker arm 24; and the cross-sectional area of a cutting portion 61 of cutting means 28. Such lengths, cross-sectional areas, and angles of rotation will in turn be dependent upon: the type of material 31 to be drilled; accessibility to that particular portion of material 31; the type of cutting means 28 used; and other factors commonly considered in drilling processes.

To enable drilling apparatus 20 to drill a curved bore having a large angle of curvature or rotation, pivot pin 52 is located as near as possible or as is desirable to a protrusion or edge 62 near an opening of housing 36. Rocker arm 24 is secured to housing 36 in such a manner that when rocker arm 24 is pivoted or rotated, second end 60 of curved guide means 26 extends and cantilevers away from housing 36, passing through a predetermined curved path. Thus, if material 31 is juxtaposed near edge 62 of housing 36, rotation or pivotal movement of rocker arm 24 will urge second end 60 of curved guide means 26 toward material 31.

The forward, cantilevered, or extended second end 60 of curved guide means 26 is provided with means 64 for securing cutting means 28 thereto. Securing means 64 may comprise an anchoring sleeve, collar, bearing, bushing, or other structure to hold and retain the leading, cutting portion 61 of cutting means 28 in place.

In the preferred form of the invention, curved guide means 26 comprises a channel 65. Curved guide means 58 is designed with elongated side walls at second end 60 that can be folded or bent over to form a collar, ring, conduit, or bore through which cutting portion 61 may pass and then be secured. In essence, the elongated side walls form a bearing surface within which cutting portion 61 may be held and/or rotated.

### Cutting Means

Cutting means 28 may comprise any apparatus or method which is capable of cutting or drilling a curved bore. Traditional and/or nontraditional machining processes may be used, including mechanical, electrochemical, and thermal processes.

More particularly, the following processes may be used for cutting means 28: a mechanical rotating drill bit or burr; abrasive flow machining (AFM); fluid or water jet machining; abrasive water jet machining; hydrodynamic machining; ultrasonic machining (impact grinding and rotary ultrasonic machining); electrochemical machining (ECM); electrolytic hole machining using an apparatus sold by General Electric Company under the trademark ELECTROSTREAM; electron beam machining (EBM); laser beam machining (LBM); electric discharge machining (EDM); plasma beam machining (PBM); and/or plasma arc cutting (PAC). The above list of processes is not to intended to be limiting. Other processes may also be used.

As shown in FIGURES 1-5, 6 and 11-20, a rotary cutting means 66 may be used. Rotary cutting means 66 may comprise: cutting portion 61; an elongated flexible drilling energy carrier or drive shaft 68; a rigid drive shaft 70; and a conventional drive motor assembly 72.

Cutting portion 61 is defined by a rotary drill bit, burr, or the like, which when rotated and urged against material 31 is capable of boring into material 31. For example, cutting portion 61 may comprise a carbide, steel, diamond, or other hard material which is attached or welded to a terminal or distal end 74 of flexible drive shaft 68.

Cutting portion 61 has cutting edges 73 (not shown) formed therein. Cutting edges 73 may be formed either before or after cutting portion 61 is attached to flexible drive shaft 68. Where flexible drive shaft 68 comprises stairless steel or other metal, a portion of distal end 74 may be melted to form a hardened nugget 76 which is integral with a remaining portion of flexible drive shaft 68. Nugget 76 may then be machined to form cutting portion 61 and cutting edges 73.

As has been explained, distal end 74 of flexible drive shaft 70 is rigidly secured to cutting portion 61. Distal end 74 is rotatably secured to housing 36 by being inserted into and held by securing means 64.

To enable passage of curved guide means 26 through the aforementioned curved path within material 31, the cross-sectional diameter of cutting portion 61 must be equal to or greater than the cross-sectional diameter of curved guide means 26. In essence,, securing means 64 urges cutting portion 61 against and through material 31. Proper alignment and path direction is provided by the curvilinear design and rotational movement of curved guide means 26.

Flexible drive shaft 68 is generally a flexible drill shaft or shank which is initially retained within enclosure 38 of housing 36. As rocker arm 24 is pivoted, cutting portion 61, distal end 74 of flexible drive shaft 68, securing means 64, and curved guide means 26 are extended outwardly from within enclosure 38 through a opening in housing 36.

Channel 65 is designed to guide, receive, and retain a forward portion 78 of flexible drive shaft 68 as such forward portion 78 is extended outwardly from within enclosure 38. As forward portion 78 is received within channel 65 flexible drive shaft 68 is urged from a first linear path to the same curvilinear path as that experienced by curved guide means 26. Once cutting portion 61 begins to drill into material 31, flexible drive shaft 68 is further retained and supported by the interior sides of the bore created within material 31.

As shown in FIGURE 1, flexible drive shaft 68 may initially move along a first direction or approach path and then be directed by curved guide means 26 to pass along a second or curvilinear path having a predetermined degree of curvature.

The first or approach path may be generally normal to a surface of material 31. Alternatively, the first approach path may be from any desired angle with respect to the surface of the material 31 to be drilled. FIGURE 20 illustrates dual flexible drive shafts 68 whose initial orientation is not normal to the surface of material 31.

FIGURE 1 illustrates a drilling apparatus 20 which will drill a curved bore that is generally located within a vertical plane. The curved bore begins within a third quadrant of a Cartesian coordinate system having its axis through pivot pin 52 and then passes upwardly into a second quadrant.

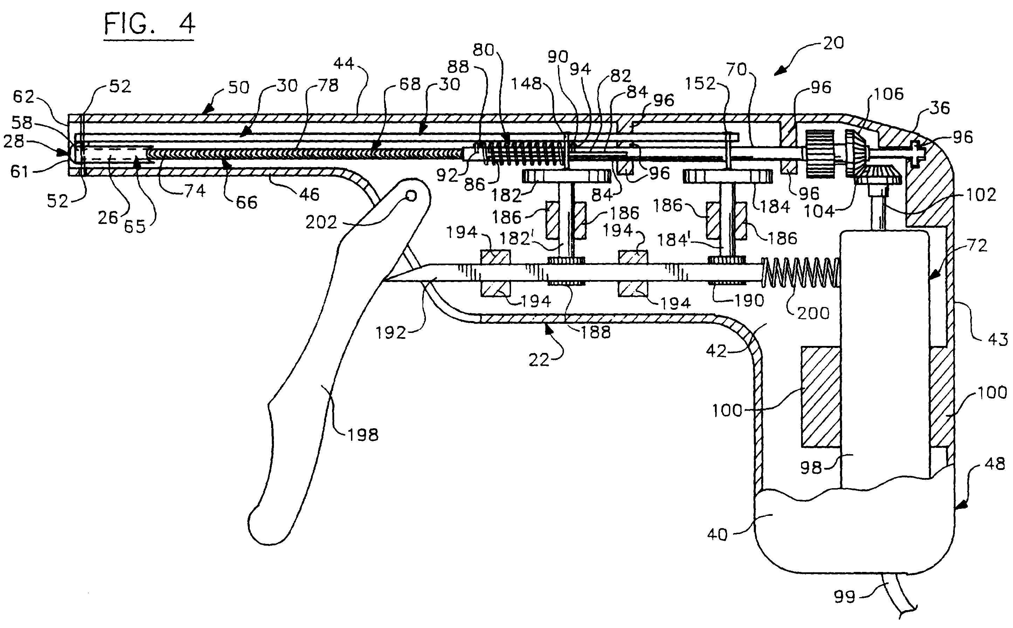

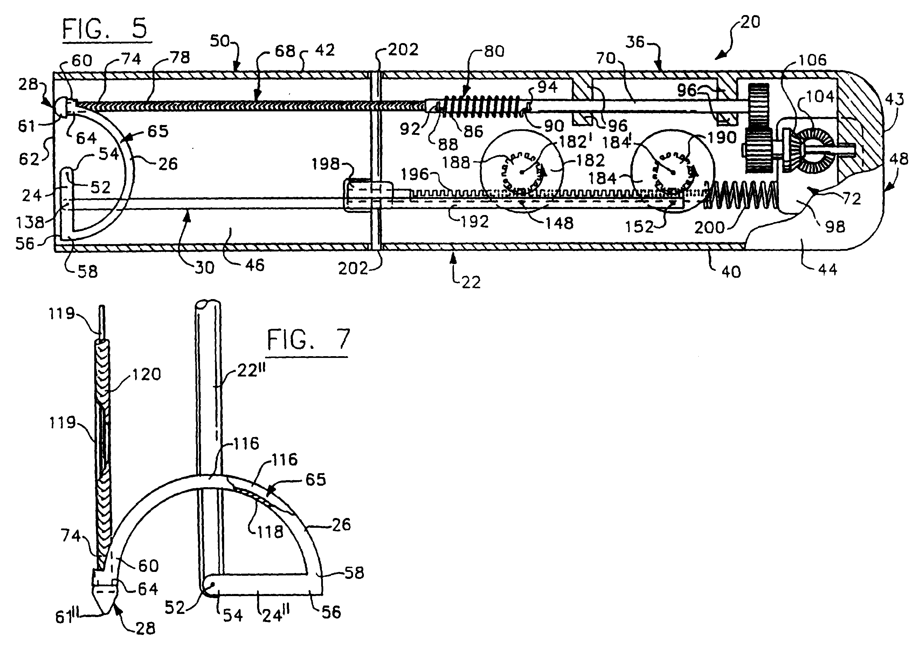

FIGURES 4 and 5 illustrate another embodiment of drilling apparatus 20 which will drill a curved bore that is generally located within a horizontal plane. The curved bore begins within a second quadrant of a Cartesian coordinate system having its axis through pivot pin 52 and then passes sideways into a third quadrant.

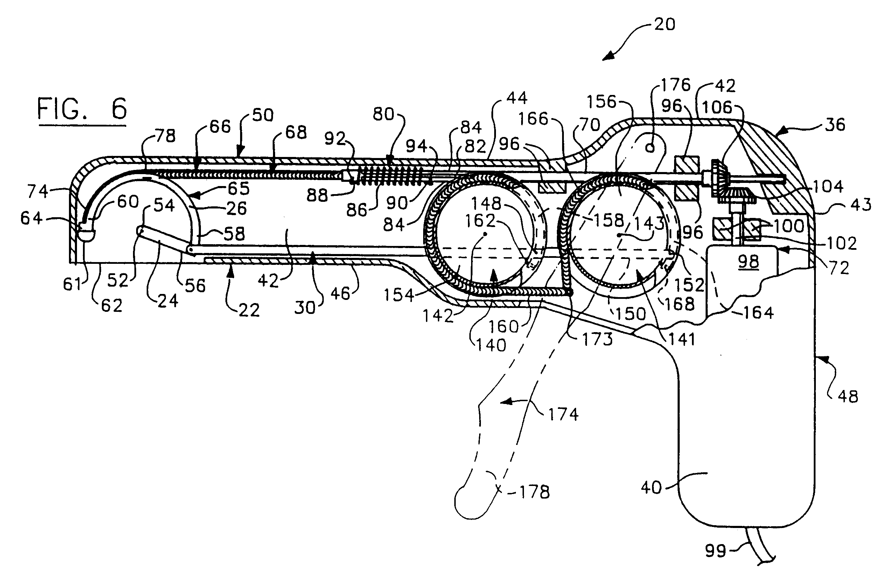

FIGURE 6 illustrates a embodiment, wherein a curved bore is generally located within a vertical plane, however, the curved bore begins within a third quadrant and passes into a fourth quadrant.

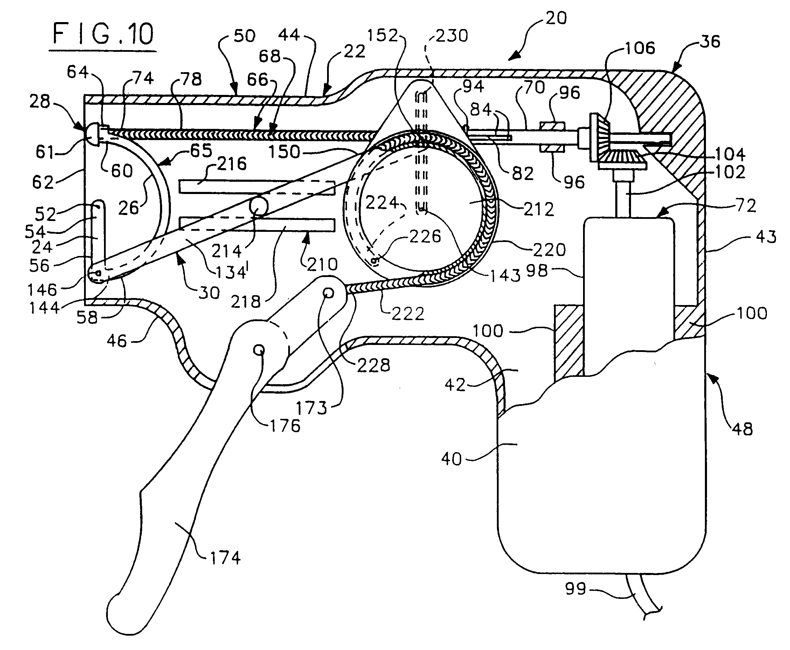

FIGURE 10 illustrates an embodiment, wherein a curved bore is formed within a vertical plane beginning within a second quadrant and passing into a third quadrant.

FIGURES 13-19 illustrate how rocker arm 24, curved guide means 26, securing means 64, and cutting portion 61 may pivot within one plane and the initial orientation or one or more flexible drive shafts 68 may be within a different plane.

Such embodiments are only illustrative. Many other orientations and cutting paths may be created using the claimed invention.

Elongated flexible drive shaft 68 is operationally connected to an elongated rigid drive shaft 70 or shank which in turn is driven by a conventional drive motor assembly. The connection of flexible drive shaft 68 to rigid drive shaft 70 may comprise any method or apparatus which allows flexible drive shaft 68 to be extended into material 31. The process of drilling the bore necessarily requires flexible drive shaft to follow the urging and direction of curved guide means 26. If flexible drive shaft 68 is not provided with sufficient length, binding might occur.

In one embodiment, drilling apparatus 20 is provided with an expandable coupling 80 which connects flexible drive shaft 68 to rigid drive shaft 70. For example, expandable coupling 80 may comprise a pair of mated male 82 and female 84 elements. Male element 82 may have a square, rectangular, triangular, star-shaped, or other cross-sectional configuration which fits into a mated slot, bore, or cannula within female element 84. The particular configuration is not necessarily important, just as long as the coupling or joint permits longitudinal expansion and still transmits rotational movement from rigid drive shaft 70 to flexible drive shaft 68.

Expandable coupling 80 may be spring-biased toward a closed, joined position. For example, a helical tension spring 86 may be provided to urge male element 82 into engaged contact with female element 84. Tension spring 86 also restricts lateral movement of flexible drive shaft 68 with respect to rigid drive shaft 70. As shown in FIGURE 1, tension spring 86 may be placed around expandable coupling 80. Tension spring 86 may also be provided with offset hooks 88 and 90 which engage corresponding eyelets 92 and 94 on flexible drive shaft 68 and on rigid drive shaft 70, respectively.

One or more mounting brackets 96 used to support rigid drive shaft 70 for rotational movement may be attached to or incorporated within housing 36. Mounting brackets 96 are shown in FIGURES 1, 4-6 and 10-12. To present a clearer illustration of other elements, mounting brackets 96 have been omitted from the remaining Figures. Collars, bearings, bushings and/or other supporting structure (not shown) may also be used to assure proper orientation, and movement of rigid drive shaft 70. For example, means 96 to adequately restrict longitudinal and lateral movement of rigid drive shaft 70 should be provided.

Drive motor assembly 72 may comprise any one of many drive motor units. A wide variety of different drive motor units are commonly used within each particular industry. The type and size of drive motor assembly 72 will depend upon the availability of parts, preference of the user, and purpose for which drilling apparatus 20 is used. Within the surgical and dental professions, hand-held drive motor units are commonly used.

In one embodiment, a drive motor assembly having a controllable, powered, pneumatically driven motor 98 is used. For surgical environments, the inventor prefers to use a compressed, nitrogen pneumatic motor. Power is supplied to motor 98 through a power cord or hose 99 which may extend outwardly from within enclosure 38 of housing 36. Alternatively, electric or hydraulic drive motors may be used.

One or more mounting brackets 100 may be used to rigidly support motor 98 to housing 36. Motor 98 has a rotatable shaft 102 whereupon an angular coupling or gear connection is provided to transfer rotational movement from rotating shaft 102 to rigid drive shaft 70. As shown in FIGURE 1, a pair of meshed bevel gears 104 and 106, having intersecting axes is rigidly attached to rotatable shaft 102 and to rigid drive shaft 70, respectively. Rotation of shaft 102 turns bevel gear 104, which in turn engages and rotates bevel gear 106 and rigid drive shaft 70. Other gear systems and angular couplings could also be used.

In another embodiment, cutting means 28 may utilize a electric discharge machining (EDM) process to create a curved bore. A drilling apparatus 20 which uses this process is illustrated in FIGURES 8 and 9.

Electric- or electro- discharge machining (EDM) cuts metal by discharging electric current stored in a capacitor bank (not shown) across a thin gap between a tool or cutting portion 61 (cathode) and material 31 (anode). Literally thousands of sparks per second are generated and each spark produces a tiny crater by melting and vaporizing the metal, thus eroding the shape of cutting portion 61 into material 31.

Cutting portion 61 may be made of a variety of materials, including: graphite, copper, brass, copper-tungsten, aluminum, 70/30 zinc tin, and other alloys. Graphite is the preferred material. The choice of material for a particular application depends upon such factors as: how easily can cutting portion 61 be machined from the material; how fast such material wears, or how susceptible the material is to spark erosion; how fast the particular material cuts; what kind of quality of finish can be produced; what type of power supply is needed and/or is available; and how much the material costs.

The distance between cutting portion 61 and the nearest surface of material 31 represents the overcut and is equal to the length of the spark. The length of the spark is essentially constant over all areas of cutting portion 61, regardless of its size or shape. Typical overcut values range from 0.0005 to 0.020 inches. Overcut depends on the gap voltage and amperage. With proper tooling, the cross-sectional dimension of cutting portion 61 is basically equal to the desired dimension of the part less the overcut value.

Material 31 may be immersed within a dielectric fluid 108 which in turn is retained within a container 109 (shown in FIGURES 8 and 9). Dielectric fluid 108 is used to: provide insulation properties between curved guide means 26 and material 31; confine the sparks within a local area immediately adjacent to cutting portion 61; serve as a conductor for the sparks between cutting portion 61 and material 31; serve as a coolant or heat bank to cool down rocker arm 24, curved guide means 26, securing means, and portions of cutting means 28; and to flush out debris located between cutting portion 61 and material 31.

Dielectric fluid 108 must ionize to provide a channel for the spark and then quickly deionized to become an insulator at further distances from cutting portion 61. Polar compounds, like glycerine-water (90:10) with triethylene oil as an additive, may be used. Alternatively, traditional cutting fluids like kerosene may be used.

Electric discharge machining (EDM) allows each spark to contain a discrete, measured, and controlled amount of energy. This enables the cutting speed and surface finish to be accurately predicted, and the size of the bore can be carefully controlled. The heat generated by the sparks melts the metal, and the impact of the spark causes the metal to be ejected, vaporized, and recast within dielectric fluid 108 as spheres. Material 31 of any hardness can be cut using this process, as long as the material can conduct electricity.

Electric discharge machining (EDM) removes almost all mechanical forces which would otherwise be required to drill a bore. Thus, fragile parts may be easily tooled with the present invention using and EDM process.

Increased controllability, versatility, and accuracy of the EDM process allows the present invention to drill curved bores in carbides, steels, and metals of any hardness, significantly reducing the cost that would otherwise be required to manufacture tools and dies to achieve a similar result.

As shown in FIGURE 7, curved guide means 26 may have an outwardly extending, arcuate, receiving channel 65 defined by sidewalls 116 and base 118. Receiving channel 65 is used to guide, receive, and retain a flexible drilling energy carrier portion of cutting means 28. Thus, cutting portion 61 may be directly connected to a remote power source (not shown).

For example, a flexible drilling energy carrier in the form of an optical conduit 119 may be enclosed within a protective insulator 120. Optical conduit is then connected to a source of laser light (not shown). As rocker arm 24 pivots about pivot pin 52, which is held in position by support structure 22'', curved guide means 26 and cutting portion 61'' are advanced toward material 31. The motion and path of curved guide means 26 cause optical conduit 119 and protective insulator 120 to be guided, received, and retained within channel 65.

Channel 65 may also be used with a rotary cutting portion 61, and flexible drive shaft 68 as shown in FIGURES 1-6.

Thus, when rocker arm 24 is pivoted, curved guide means guides and advances cutting means 28 away from support structure 22 to bore a hole along a predetermined curved path within material 31.

After the bore has been created, the motion of rocker arm 24 is reversed and curved guide means 26 and cutting means 28 are removed from within material 31. The insertion and retraction of curved guide means 26 and cutting means 28 into and from material 31 is defined by the oscillation of rocker arm 24.

When rocker arm 24, curved guide means 26, and cutting portion 61 of cutting means 28 lie within a common plane, curved bores may be created which have up to a 180 degree rotation. In other words, the length of the curved bore may generally be determined by multiplying the length of rocker arm 24 between pivot pin 52 and the center of the rigidly attached curved guide means 26 by a value of π.

Alternatively, rocker arm 24, curved guide means 26, and cutting portion 61 may define a helical drilling apparatus 20 which forms a cylindrical spiral bore. This may be accomplished by securing a helically shaped curved guide means (not shown) to the extended second end 56 of rocker arm 24. Cutting portion 61 is secured to the cantilevered second end 60 of curved guide means 26. This design enables rocker arm 24 to be pivoted more than 360 degrees and to actually serve as a crank, screwing cutting portion 61 and curved guide means 26 into the material. Of course, rocker arm 24 would necessarily have to move along its axis of rotation towards material 31 as curved guide means 26 is inserted into material 31.

### Actuation Means

Pivotal rocker arm 24, curved guide means 26, and cutting means 28 may be remotely actuated by an actuation means which simply is a drill guide advancing. mechanism. Such actuation means selectively urges rocker arm 24 to pivot or rotate, thereby urging curved guide means 26 and cutting means 28 to advance toward material 31 and bore a hole along a predetermined curved path within material 31. Such actuation means may also be used to retract rocker arm 24 to an initial position and thereby withdraw curved guiding means 26 and cutting means 28 from within material 31. The insertion and retraction of curved guide means 26 and cutting means 28 into and from material 31 defines an oscillation of rocker arm 24.

Pivotal rocker arm 24, curved guide means 26, and cutting means 28 may be remotely actuated by a wide variety of actuation means 30.

As illustrated in FIGURES 8 and 9, actuation means 30 may simply comprise tension or compression spring 122 having a first end or arm 124 and a second end or arm 126. First end or arm 124 is urged against rocker arm 24. Second end or arm 126 is urged against support structure 22. Thus, the tensile or compression forces stored within spring 122 urge spring 122 either open or closed, depending upon whether spring 122 is a compression or tension spring. In either case, the forces

stored within spring 122 should urge rocker arm 24, curved guide means 26, and cutting means 28 to rotate about pivot pin 52.

In one embodiment, spring 122 is a tension spring which urges cutting means 28 toward and/or against material 31. During operation, rocker arm 24 is pivoted clockwise as shown by arrow 128. This raises cutting portion 61 above an upper surface of material 31. Manual forces exerted on rocker arm 24 are then released so that spring 122 may pivot rocker arm 24 in a counter-clockwise direction, thereby urging cutting portion 61 into close proximity with the upper surface of material 31.

Additional elements may be used to enable cutting portion 61 to be retracted from within material 31 without necessitating that an operator reach into dielectric fluid 108 to rotate rocker arm 24 in a clockwise direction. For example, first end 54 of rocker arm 24 may be rigidly attached to a first end of an elongated pivot pin 52. Pivot pin 52 may be protected and insulated within an outer sheathing 129. Pivot pin 52 is then rotatably secured to support structure 22. A lever or crank 130 is rigidly attached to a second end of pivot pin 52. Rigid attachment may be accomplished by any appropriate means. In FIGURE 10, nut 131 and washers 132 are threaded onto the first and second ends of pivot pin 51. Other means of attachment, such as by welding, may also be used.

Rotation of crank 130 causes pivot pin 52 to rotate within support structure 22, and thereby urge rocker arm 24 to rotate in a similar direction. Rotation of rocker arm 24 causes curved guide means 26 and cutting means 28 to advance or retract, depending upon the direction of rotation, through the predetermined curved path.

Other forms of actuation means 30 may also be used. For example, FIGURES 1-6,10-12, 13-15, 16-18, and 19 illustrate different types of connecting rods 134 and push/pull flexible linkages 136 that may be pivotally secured to either rocker arm 24 or to curved guide means 26. Preferably, connecting rods 134 or flexible linkages 136 are secured to rocker arm 24 at a juncture or intersection of rocker arm 24 with curved guide means 26. Alternatively, as shown in FIGURE 5, connecting rod 134 may be connected to rocker arm 24 at a intermediate point 138 between first end 54 and second end 56.

Appropriate movement of connecting rod 134 or flexible linkage 136 causes rocker arm 24, curve guide means 26, and cutting means 28 to advance or retract through a predetermined curved path.

FIGURES 1-3 illustrate a first embodiment of the present invention, wherein actuation means 30 comprises a double parallel-crank mechanism. Rocker arm 24, and two different crank systems 140 and 141 are rotationally secured to support structure 22, in such a manner that their axes are located along a common ray or line. First crank system 140 rotates about a pivot pin 142. Second crank system 141 rotates about a pivot pin 143.

A single connecting rod 134 is connected to rocker arm 24 and to each of the two crank systems 140 and 141. A first end 144 of connecting rod 134 is pivotally secured to second end 56 of rocker arm 24. A pivot pin 146 is used to secure connecting rod 134 to rocker arm 24.

Connecting rod 134 is pivotally secured to a first crank system 140 at a intermediate location along the length of connecting rod 134. Pivot pin or rivet 148 may be used to secure connecting rod 134 to first crank system 140. The distance between pivot pin 142 and pivot pin 148 is substantially equal to the distance between pivot pin 52 and pivot pin 146.

A second end 150 of connecting rod 134 is pivotally connected to second crank system 141 by means of a pivot pin 152. Similarly, the distance between pivot pin 143 and pivot 152 is substantially equal to the distance between pivot pins 52 and 146, and between pivot pins 142 and 148.

The distance between pivot pins 146 and 148 is substantially equal to the distance between pivot pins 52 and 142. Likewise, the distance between pivot pins 148 and 152 is substantially equal to the distance between pivot pins 142 and 143.

In essence, support structure 22, rocker arm 24, connecting rod 134, first crank system 140, and second crank system 141 move within parallel planes and form a plane parallelogram.

Connecting rod 134, first crank system 140, and second crank system 141 may be of any form or configuration so long as such elements perform the described function and do not interfere with the desired motion.

Connecting rod 134 is capable of rotating freely about pivot pins 146,148, and 152, even through a complete rotation of 360 degrees if need be. In most cases, however, rotation will be limited to 180 degrees.

Thus, the cutting means 28 is first moved out of the housing along the arcuate path defined by swinging of the curved guide means. When the angle of rotation reaches about 90°, the cutting means moves back towards the housing as the curved guide means continues its rotation caused by the translating motion imported by the push rod 134.

Movement of either first crank system 140 or of second crank system 141 will cause connecting rod 134 to transmit substantially identical rotational movement to rocker arm 24.

Means for rotating first and/or second crank systems 140 and 141 are also provided.

First and/or second crank systems 140 and 141 may have one or more pulleys incorporated therein. Such pulleys share the similar axis of rotation as their corresponding crank system. As shown in Figures 1-3, first and second crank systems 101 and 141 comprise pulleys 154 and 156, respectively.

Pulleys 154 and 156 have outwardly extending channels of substantially similar diameter within which one or more flexible tension cords, chains, cables, or the like may be placed. The pulleys or flexible tension cords provides smooth, easy actuation and rotation of the plane parallelogram, attached curved guide means 26, and cutting means 28.

As shown in Figure 1, a first end 158 of a first tension cord 160 is secured pulley 154 by fastener 162. Similarly, a first end 164 of a second tension cord 166 is secured to pulley 156 by fastener 168. Flexible tension cords 160 and 166 run within the exterior channels of pulleys 154 and 156, respectively. A second end 170 of first tension cord 160 and/or a second end 172 of second tension cord 164 are rigidly secured by a fastener 173 to an easily controlled trigger 174, lever, switch, or the like.

Trigger 174 extends outwardly from within enclosure 38 and is pivotally secured by pivot pin 176 to support structure 22. Pivot pin 176 is positioned in such a manner as to give mechanical advantage to an extended portion 178 of trigger 174.

Pivotal movement of extended portion 178 about pivot pin 176 causes fastener 173 to move through a predefined arc or path which is defined by the angle of rotation and radial distance between pivot pin 176 and fastener 173. Movement of fastener 173 causes tension cords 160 and 166 to move longitudinally, which in turn rotate pulleys 154 and 156. Pulleys 154 and 156 are rotated at substantially the same rate, thereby causing connecting rod 134 to rotate rocker arm 24, curved guide means 26, and cutting means 28.

A tension spring 180 may be operationally secured to support structure 22 to urge first and/or second crank systems 140,141 toward rotation or to retract crank systems 140 and. 141 from an earlier rotation. One end of tension spring 180 may be secured to pivot pin 143, which in turn is rigidly fixed to support structure 22. The other end of tension spring 180 may be secured to pulley 156.

It is important to note that at its critical position, where connecting rod 134 and pivot pins 52, 142 and 143 all lie within a substantially similar ray or line, flexible tension cord 160 continues to exert a force upon first crank system 140, and tension cord 166 exerts a force upon second crank system 141. Because of this design, actuation means 30 has no uncertainty of motion at such a position. In other words, there are no dead points.

FIGURE 6 illustrates an alternative position for trigger 174, pivot pin 176, and fastener 173. As can be seen, the rotational direction of first and second crank systems 140 and 141 depend upon the desired motion for actuation and path of rocker arm 24.

In a second embodiment of the present invention, as illustrated in FIGURES 4-5, the pulleys 154 and 156, and tension cords 160 and 166 of the first embodiment are replaced with a rack and pinion system. In this second embodiment, first crank system 140 and second crank system 141 comprise disks 182 and 184 which are connected to connecting rod 134 by pivot pins 148 and 152, respectively. Disk 182 is rigidly secured to a shaft 182'. Shaft 182' is rotationally secured to support structure 22 by brackets 186. Disk 184 is rigidly secured to a shaft 184'. Shaft 184' is also rotationally secured to support structure 22 by brackets 186. A first pinion 188 is rigidly secured to shaft 182' of first crank system 140. A second pinion 190 is rigidly secured to shaft 184' of second crank system 141.

A rack 192 is located within enclosure 38 and is secured to support structure 22 in such a way as to enable rack 192 to slide back and forth within fixed guides 194. Rack 192 has rack teeth 196 which mesh with pinions 188 and 190. When rack 192 slides within fixed guides 194, rack teeth 196 cause pinions 188 and 190, shafts 182' and 184', and disks 182 and 184 to rotate, which in turn drive connecting rod 134. Movement of connecting rod 134 causes rocker arm 24, curved guide means 26, and cutting means 28 to pivot within their predetermined curved path.

Rack 192 is held in operable contact with a lever 198 by a compression spring 200. Lever 198 pivots about a pivot pin 202.

In a further embodiment, illustrated in FIGURES 10-12, actuation means 30 may comprise a crossed-crank mechanism, having: a rotatable disk crank 212; a levered connecting rod 134'; a guide 210; and trigger 174.

Disk crank 212 is rotatably secured to support structure 22 and pivots about pivot pin 143.

A first end 144 of levered connecting rod 134' is secured to rocker arm 24 by pivot pin 146. A second end 150 of levered connecting rod 134' is secured to rotating disk crank 212 by pivot pin 152.

Levered connecting rod 134' is also provided with a slidable slide or pivot pin 214 at some intermediate point between first end 144 and second end 150. Preferably, slidable pivot pin 214 is located at a midpoint between first end 144 and second end 150.

Slidable pivot pin 214 is placed within a guide 210 having an upper portion 216 and a lower portion 218. As slidable pivot pin 214 slides within guide 210, upper portion 216 and lower portion 218 serve as fulcrums against which levered, connecting rod 134' is urged. The fulcrums are defined by interior side walls of a groove, slot, or channel of guide 210 formed within or secured to support structure 22. The fulcrums provide a rigid point of support about which connecting rod 134' may pivot.

Similar to the explanation given above, disk crank 212 may be provided with a pulley 220 which rotates about pivot pin 143. Pulley 220 has an outwardly extending channel within which a flexible tension cord 222 may be received, guided, and stored.

Flexible tension cord 222 has a first end 224 which is attached to pulley 220 by a fastener 226. A second end 228 of flexible tension cord 222 is attached to trigger 174 by fastener 173. Trigger 174 pivots about a pivot pin 176 to impart a tension and movement within cord 222. As a result of the movement of trigger 174, disk crank 212 is rotated.

The distance between pivot pins 52 and 146 is substantially similar to the distance between pivot pins 143 and 152.

In one embodiment (not shown), the distance between pivot pins 146 and 152 is substantially equal to the distance between pivot pins 52 and 143. Were this so, a true crossed-crank mechanism would be created.

In an alternative embodiment, shown in FIGURES 10-12, a modified crossed-crank mechanism is illustrated. Generally, a slot 230 is provided within disk crank 212, and pivot pin 152 is free to slide within slot 230. Disk crank 212 is also elongated adjacent to slot 230 to provide support and clearance for slot 230. This design permits a lesser tolerance to be used between the various parts of the apparatus.

As trigger 174 is pivoted about pivot pin 176, fastener 173 pulls on tension cord 222 which in turn rotates disk crank 212. As disk crank 212 rotates, pivot pin 152 is allowed to freely slide within slot 230. Tangential forces upon pivot pin 152 force connecting rod 134' to rotate about pivot pin 214. Pivot pin 214 is also able to freely slide within guide 210. Guide 210 serves as a fulcrum against which connecting rod 134' is urged. Further rotational movement of disk crank 212 is transmitted through connecting rod 134' to cause rocker arm 24 to move with generally the same motion as disk crank 212, except in an opposite rotational direction.

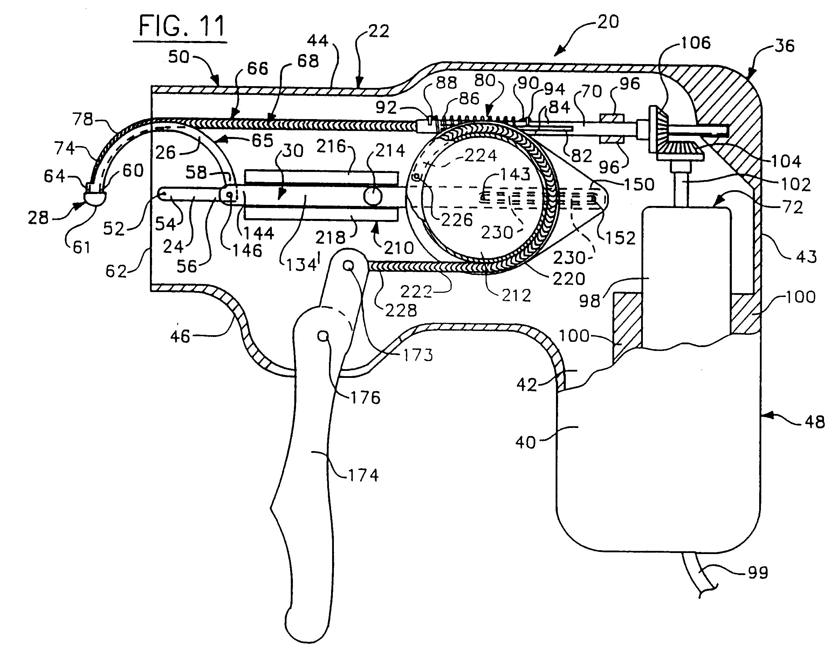

FIGURE 11 illustrates this embodiment with a partially extended condition. Further rotation of disk crank 212 forces connecting rod 134' to push against pivot pin 146 and force rocker arm further upward within its predetermined curved path.

Similar to the above description, a spring (not shown) may be used to urge disk crank 212 toward rotation or toward a retraction from an earlier rotation. The spring should be operationally secured between support structure 22 and disk crank 212.

In still further embodiments, as shown in FIGURES 9, 13-19, multiple rocker arms 24, multiple curved guide means 26, multiple cutting means 28, and multiple actuation means 30 may be used in unison or within a single apparatus.

FIGURE 9 illustrates many drilling apparatus 20 being used in unison.

FIGURES 13-19 illustrate multiple drilling apparatus used within a single apparatus.

More particularly, Figure 15 illustrates how two off-centered pivot points 52 may be used to form a wish-bone shaped bore.

Of particular interest is how a flexible push/pull linkage 300 may be used actuate the rotational movement of rocker arm 24. Appropriate guides 302 are provided to properly direct the forces transmitted within flexible linkage 300.

The present invention also allows cutting means 28 to have a wide range of orientations with respect to bore surface of material 31. This is accomplished by controlling the path and angle of rotation of curved guide 26 and cutting means 28, and by placing curved guide means 26 and cutting means 28 at various angles with respect to the remaining elements of drilling apparatus 20 and/or with respect to material 31 to be drilled.

Figure 18 illustrates a mechanism which has an actuating means 30 similar to that described in the U.S. patent 4,941,466. The differences, however, accentuate the fact that a linear to a curvilinear path for the flexible drill shaft is not necessary as required. These drawings depict only a few of several different types of apparatus that may be created wherein the flexible drill shaft passes from a curvilinear path to a curvilinear path.

Thus, with the present invention an incision may be made at a considered safe location and then the drilling means may be appropriately positioned near or against the, bone structure to be drilled. Once in position, the drilling may take place without having to move or manipulate the position of drilling apparatus 20.

The means and construction disclosed herein are by way of example and comprise primarily the preferred form of putting the invention into effect. Although the drawings depict a preferred and alternative embodiments of the invention, other embodiments have been described within the preceding text. One skilled in the art will appreciate that the disclosed device may have a variety of shapes and configurations. Additionally, persons skilled in the art to which the invention pertains might consider the foregoing teachings in making various modifications, other embodiments, and alternative forms of the invention.

### Industrial Applicability

The present invention may be used within a wide variety of industries, wherein simple, reliable, easily used apparatus and methods are needed to form one or more curved bores within a solid material. The apparatus of this invention is also compact, functional, unobtrusive, efficient, reusable, durable, rugged, is easily constructed, and is inexpensive and economical to manufacture. Traditional or nontraditional drilling processes may be used. The present invention not only increases the speed and simplifies the procedure to form curved bores, it also provides a drilling apparatus which requires less access room for operation and does not damage adjacent material. This invention also allows drilling to be initiated from a wide range of orientations with respect to the bored surface.

Although the invention has a wide range of applications, the invention has special application in surgical procedures, wherein a ligament, tissue, wire, or other element must be secured to a bone surface. The present invention permits such procedures to be accomplished in areas of extremely limited access. The curved bore may be produced even within a very small, deep access opening or incision, with minimum damage to adjacent bone structure and soft tissue. One the curved bore is formed, a suture or other attaching filament may be easily passed through the bore to anchor the tissue or ligament to the bone.

## Claims

1. An apparatus for drilling a curved bore within a material comprising:
(a) a support structure (22);
(b) a rocker arm (24) pivotally secured to said support structure (22) at a pivot point (52), said pivot point defining an axis of rotation, said rocker arm (24) defining a rotating link or crank which rotates or oscillates about said pivot point (52), said rocker arm having a first end (54) located near said pivot point (52) and having a second end (56) extending radially outward from said pivot point (52);
(c) curved guide means (26) having a first end (58) rigidly attached to or integral with said second end (56) of said rocker arm (24), said curved guide means (26) having an extended second end (60) which cantilevers outwardly from said rocker arm (24), said curved guide means (26) generally laying within an arc defined by a rotational path of said second end (56) of said rocker arm (24) as said rocker arm (24) is rotated about said pivot point (52), said pivot point (52) being located near a protrusion or edge of said support structure (22);
(d) means (28) for cutting said material within confines of said curved bore;
(e) means (64) for securing said cutting means (28) to said second end (60) of said curved guide means (26);
(f) means (30) for remotely actuating rotation of said rocker arm (24), and
the cutting means (28) comprising a cutting head and a flexible drilling energy carrier (68) for connection to a remote power source said cutting head being adapted to advance and retract through a predetermined path first outward away from the support structure then at least partially inward toward the support structure, and characterised by the curved guide means (26) having an outwardly extending receiving channel (65) into which said flexible drilling energy carrier (68) of said cutting means (28) is guided, received and retained, said channel (65) receiving, retaining and releasing said flexible drilling energy carrier (68) as said cutting means (28) and said curved guide means (26) is advanced and retracted to drill a curved bore through said material,
and in that said actuation means (30) comprises a connecting rod or push/pull flexible linkage pivotally secured to either said rocker arm or to said curved guide means, movement of said connecting rod or linkage causing said rocker arm, said curved guide means, and said cutting head to advance or retract through said predetermined path.

2. The apparatus of claim 1, wherein the flexible drilling energy carrier (68) is mounted in the support structure (22) for linear movement toward and away from the curved guide means (26).

3. The apparatus of any preceding claim, wherein the support structure (22) comprises a hand held housing unit (36).

4. The apparatus of any preceding claim, wherein the cutting means (28) incorporates therein a mechanical, electrochemical or thermal machining process.

5. The apparatus of any preceding claim, wherein the actuating means (28) comprises at least one crank system (140 or 141).

6. The apparatus of any preceding claim, wherein the actuating means (30) translates the entire connecting rod (134) along an arcuate path.

7. The apparatus of any preceding claim, in which the actuating means includes at least two crank systems (140,141), the connecting rod (134) being pivotally connected to both of the crank systems for movement of each point of the connecting rod along a circular path.

8. The apparatus of any preceding claim, in which the actuating means (30) moves the cutting means (28) through an angle of about 180°.

9. The apparatus of any preceding claim, wherein the actuating means (30) further comprises a lever or trigger pivotally secured to the support structure (22), the connecting rod (134) being operationally secured to said lever in such a manner that movement of said lever causes the push rod to translate.

10. The apparatus of any preceding claim, wherein the actuating means (30) further comprises a meshed rack and pinion, longitudinal movement of said rack (192) causing said pinion (188) to rotate which in turn causes said connecting rod (134) to translate.

11. The apparatus of any preceding claim, wherein the connecting rod (134) is secured to the rocker arm (24) or the curved guide means (26) at a juncture between the rocker arm and the curved guide means.

12. The apparatus of any preceding claim, wherein the actuating means (30) comprises a double parallel-crank mechanism (140,141).

13. The apparatus of claim 12, wherein said double parallel-crank mechanism comprises:
(a) said support structure (22);
(b) said rocker arm (24) pivotally secured to said support structure at a fixed pivot point;
(c) a first crank system (140) rotationally secured to said support structure;
(d) a second crank system (141) rotationally secured to said support structure;
(e) said connecting rod (134) having a first end, an intermediate point located along a length of said connecting rod, and a second end, said first end of said connecting rod being pivotally secured to said rocker arm (24), said first crank system being pivotally connected to said connecting rod at said intermediate point, said second end of said connecting rod being pivotally connected to said second crank system, said support structure, said rocker arm, said connecting rod, said first crank system, and said second crank system forming a plane parallelogram; and
(f) means for rotating said first and second crank systems, said rotating means being secured to at least first crank system.

14. The apparatus of claim 13, wherein said rotating means comprises at least one tension cord (154) secured to said first crank system (140), longitudinal movement of said tension cord causing said first crank system to rotate which in turn causes said connecting rod (134) to rotate said rocker arm (24) and said second crank system (141).

15. The apparatus of claim 14, wherein said first crank system (140) comprises at least one pulley (154), said first crank system and said pulley having a similar axis of rotation, said tension cord (154) being passed around said pulley.

16. The apparatus of claim 14, further comprising a lever or trigger (174) pivotally secured to the support structure (22), the tension cord (154) being operationally secured to said lever in such a manner that movement of said lever causes the tension cord to move longitudinally and thereby rotate the first crank system (140).

17. The apparatus of claim 12, further comprising a spring operationally secured to said support structure (22), said spring urging said first or said second crank system (140 or 141) to rotate or to retract from an earlier rotation.

18. A method for forming a curved bore by the use of apparatus according to any preceding claim, excluding any method with the meaning of Article 52(4) EPC.

19. A method for drilling a curved bore within a material, which method comprises: moving an elongate flexible drilling energy carrier (68) along a first direction or approach path toward and away from a curved guide means (26), said guide means (26) being carried by a rocker means (24) generally lying within an arc defined by a rotational path centered about an axis of rotation; and remotely actuating rotation of the curved guide means (26) by translating a connecting rod (134) pivotally connected to the curved guide means along an arcuate path of an extent of more than 90°, and diverting the flexible drilling energy carrier (68) by engagement with the curved guide means (26) to pass along a curvilinear path having a curvature of more than 90° defined by the rotational path centered about the axis of rotation, while supplying drilling energy along the elongated flexible drilling energy carrier (68) to a cutting head (61) secured to said curved guide means (26) and disposed at an end of the drilling energy carrier (68), and thereby advancing the cutting head (61) toward the material to bore a hole along the predetermined curved path within the material and retracting the cutting head (61) to an initial position thereby withdrawing the curved guide means (26) and the cutting head (61) from within the material; but excluding any method within the meaning of Article 52(4) EPC.

## Patentansprüche

1. Vorrichtung zum Bohren einer gekrümmten Bohrung in einem Material, umfassend:
(a) eine Halterung (22);
(b) einen Schwenkarm (24), der schwenkbar an der Halterung (22) an einem Schwenkpunkt (52) angebracht ist, wobei der Schwenkarm (24) ein Drehverbindungsglied oder eine Kurbel bildet, die sich um den Schwenkpunkt (52) dreht oder um diesen schwenkt, wobei der Schwenkarm ein erstes Ende (54) in der Nähe des Schwenkpunkts (52) und ein zweites Ende (56) radial außen bezüglich des Schwenkpunkts (52) besitzt;
(c) eine Kurvenführung (26) mit einem ersten Ende (58), welches starr oder einstückig mit dem zweiten Ende (56) des Schwenkarms (24) verbunden ist, wobei die Kurvenführung (26) ein gestrecktes zweites Ende (60) aufweist, welches von dem Schwenkarm (24) freitragend nach außen wegsteht, wobei die Kurvenführung (26) etwa auf einem Bogen liegt, der definiert wird durch einen Drehweg des Zweiten Endes (56) des Schwenkarms (24), wenn dieser um den Schwenkpunkt (52) gedreht wird, wobei der Schwenkpunkt (52) sich in der Nähe eines Vorsprungs oder einer Kante der Halterung (22) befindet;
(d) eine Vorrichtung (28) zum Schneiden des Materials innerhalb von Grenzen der gekrümmten Bohrung;
(e) eine Einrichtung (64) zum Sichern der Schneidvorrichtung (28) an dem zweiten Ende (60) der Kurvenführung (26);
(f) eine Einrichtung (30) zur Fern-Drehbetätigung des Schwenkarms (24), wobei
die Schneidvorrichtung (24) einen Schneidkopf und einen flexiblen Bohrenergieträger (68) zur Verbindung mit einer entfernten Antriebsquelle aufweist, von denen der Schneidkopf dazu ausgebildet ist, sich über eine vorbestimmte Bahn zunächst aus der Halterung heraus und anschließend zumindest teilweise in Richtung der Halterung nach innen vor- und zurückzubewegen, **dadurch gekennzeichnet**, daß die Kurvenführung (26) einen sich nach außen erstreckenden Aufnahmekanal (65) aufweist, in dem der flexible Bohrenergieträger (68) der Schneidvorrichtung (28) geführt, aufgenommen und gehalten wird, wobei der Kanal (65) den flexiblen Bohrenergieträger ((68) aufnimmt, hält und freigibt, wenn die Schneidvorrichtung (28) und die Kurvenführung (28) zum Bohren einer gekrümmten Bohrung durch das Material vorgerückt und zurückgezogen werden,
und daß die Betätigungseinrichtung (30) eine Verbindungsstange oder eine flexible Druck/Zug-Verbindung aufweist, die schwenkbar an dem Schwenkarm oder an der Kurvenführung angebracht ist, wobei eine Bewegung der Verbindungsstange oder der Verbindung den Schwenkarm, die Kurvenführung und den Schneidkopf veranlaßt, über den vorbestimmten Weg vorzurücken oder zurückzugehen.

2. Vorrichtung nach Anspruch 1, bei der der flexible Bohrenergieträger (68) in der Halterung (22) für eine Linearbewegung auf die Kurvenführung (26) zu und von dieser weg gelagert ist.

3. Vorrichtung nach einem vorhergehenden Anspruch, bei der die Halterung (22) eine in der Hand haltbare Gehäuseeinheit (36) aufweist.

4. Vorrichtung nach einem vorhergehenden Anspruch, bei der die Schneidvorrichtung (28) einen mechanischen, einen elektrochemischen oder einen thermischen materialabhebenden Prozeß ausführt.

5. Vorrichtung nach einem vorhergehenden Anspruch, bei der die Betätigungsvorrichtnng (28) mindestens ein Kurbelsystem (140 oder 141) aufweist.

6. Vorrichtung nach einem vorhergehenden Anspruch, bei der die Betätigungseinricbtung (30) die gesamte Verbindungsstange (134) entlang einem bogenförmigen Weg verlagert.

7. Vorrichtung nach einem vorhergehenden Anspruch, bei der die Betätigungseinrichtung mindestens zwei Kurbelsysteme (140, 141) aufweist, wobei die Verbindungsstange (134) schwenkbar mit beiden Kurbelsystemen gekoppelt ist zwecks Bewegung jedes Punkts der Verbindungsstange entlang einer kreisförmigen Bahn.

8. Vorrichtung nach einem vorhergehenden Anspruch, bei der die Betätigungseinrichtung (30) die Schneidvorrichtung (28) über einen Winkel von etwa 180° bewegt.

9. Vorrichtung nach einem vorhergehenden Anspruch, bei der die Betätigungseinrichtung (30) außerdem einen Hebel oder Auslöser aufweist, der schwenkbar an der Halterung (22) angebracht ist, wobei die Verbindungsstange (134) betrieblich mit dem Hebel in der Weise gekoppelt ist, daß eine Bewegung des Hebels eine Verlagerung der Druckstange veranlaßt.

10. Vorrichtung nach einem vorhergehenden Anspruch, bei der die Betätigungseinrichtung (30) außerdem eine kämmende Zahnstangen-Ritzel-Anordnung aufweist, wobei eine Längsbewegung der Zahnstange (192) das Ritzel (188) zum Drehen bringt, was wiederum eine Verlagerung der Verbindungsstange (134) veranlaßt.

11. Vorrichtung nach einem vorhergehenden Anspruch, bei der die Verbindungsstange (134) an dem Schwenkarm (24) oder der Kurvenführung (26) an einem Verbindungspunkt angebracht ist, der sich zwischen dem Schwenkarm und der Kurvenführung befindet.

12. Vorrichtung nach einem vorhergehenden Anspruch, bei der die Betätigungseinrichtung einen Doppel-Parallelkurbelmechanismus (140, 141) aufweist.

13. Vorrichtung nach Anspruch 12, bei der der Doppel-Parallelkurbelmechanismus umfaßt:
(a) die Halterung (22);
(b) den Schwenkarm (24), der schwenkbar an der Halterung an einem festen Schwenkpunkt angebracht ist;
(c) ein erstes Kurbelsystem (140), welches drehbar an der Halterung angebracht ist;
(d) ein zweites Kurbelsystem (141), welches drehbar an der Halterung angebracht ist;
(e) wobei die Verbindungsstange (134) ein erstes Ende, einen sich an einer Stelle entlang der Verbindungsstange befindlichen Zwischenpunkt und ein zweites Ende aufweist, von denen das erste Ende der Verbindungsstange schwenkbar mit dem Schwenkarm (24) verbunden ist, das erste Kurbelsystem schwenkbar mit der Verbindungsstange an dem Zwischenpunkt verbunden ist, das zweite Ende der Verbindungsstange schwenkbar mit dem zweiten Kurbelsystem verbunden ist, wobei die Halterung, der Schwenkarm, die Verbindungsstange, das erste Kurbelsystem und das zweite Kurbelsystem ein flächiges Parallelogramm bilden; und
(f) eine Einrichtung zum Drehen des ersten und des zweiten Kurbelsystems, wobei die Dreheinrichtung zumindest an dem ersten Kurbelsystem angebracht ist.

14. Vorrichtung nach Anspruch 13, bei dem die Dreheinrichtung mindestens eine Zugschnur (154) aufweist, welche an dem ersten Kurbelsystem (140) befestigt ist, wobei eine Längsbewegung der zugschnur veranlaßt, daß sich das erste Kurbelsystem dreht, was wiederum die Verbindungsstange (134) veranlaßt, den Schwenkarm (24) und das zweite Kurbelsystem (141) zu drehen.

15. Vorrichtung nach Anspruch 14, bei der das erste Kurbelsystem (140) mindestens eine Riemenscheibe (154) aufweist, wobei das erste Kurbelsystem und die Riemenscheibe eine ähnliche Drehachse besitzen und die Zugschnur (154) um die Riemenscheibe geführt ist.

16. Vorrichtung nach Anspruch 14, weiterhin umfassend einen Hebel oder Auslöser (174), der schwenkbar an der Halterung (22) gelagert ist, wobei die Zugschnur (154) betrieblich mit dem Hebel in der Weise verbunden ist, daß eine Bewegung des Hebels die Zugschnur veranlaßt, sich in Längsrichtung zu bewegen und dadurch das erste Kurbelsystem (140) zu drehen.

17. Vorrichtung nach Anspruch 12, weiterhin umfassend eine betrieblich mit der Halterung (22) gekoppelte Feder, die das erste oder das zweite Kurbelsystem (140 oder 141) beaufschlagt, um es zu drehen, oder um es aus einer früheren Drehung zurückzuziehen.

18. Verfahren zum Ausbilden einer gekrümmten Bohrung durch Verwendung der Vorrichtung nach einem vorhergehenden Anspruch, ausgeschlossen jegliches Verfahren im Sinne des Artikels 52(4) EPÜ.

19. Verfahren zum Bohren einer gekrümmten Bohrung in einem Material, umfassend: Bewegen eines länglichen flexiblen Bohrenergieträgers (68) entlang einer ersten Richtung oder eines Annäherungswegs in Richtung auf eine und fort von einer Kurvenführung (26), wobei die Führung (26) von einer Schwenkvorrichtung (24) getragen wird, die etwa auf einem Bogen liegt, der definiert wird durch einen bezüglich einer Drehachse zentrierten Drehweg; und Fern-Drehbetätigen der Kurvenführung (26) durch Verlagern einer Verbindungsstange (134), die drehbar mit der Kurvenführung verbunden ist, entlang einer gekrümmten Bahn über eine Erstreckung von mehr als 90°, und Ablenken des flexiblen Bohrenergieträgers (68) durch Zusammenwirken mit der Kurvenführung (26), damit er sich entlang einem gekrümmten Weg mit einer Krümmung von mehr als 90° bewegt, definiert durch den bezüglich der Drehachse zentrierten Drehweg, wahrend Bohrenergie über den länglichen flexiblen Bohrenergieträger (68) einem Schneidkopf (61) zugeleitet wird, der an der Kurvenführung (26) festgelegt ist und sich an einem Ende des Bohrenergieträgers (68) befindet, um dadurch den Schneidkopf (61) in Richtung auf das Material vorzurücken und ein Loch entlang dem vorbestimmten gekrümmten Weg in dem Material zu bohren und den Schneidkopf (61) zu einer Ausgangsstellung zurückzuziehen und dadurch die Kurvenführung (26) und den Schneidkopf (61) aus dem Material zurückzuziehen, ausgeschlossen jegliches Verfahren im Sinne des Artikels 52(4) EPÜ.

## Revendications

1. Appareil pour percer un perçage courbe dans une matière, comprenant:
(a) une structure de support (22);
(b) un bras pivotant (24) fixé de façon pivotante sur ladite structure de support (22), au droit d'un point de pivot (52), ledit point de pivot définissant un axe de rotation, ledit bras pivotant (24) définissant un élément tournant ou une manivelle qui tourne ou oscille autour dudit point de pivot (52), ledit bras pivotant ayant une première extrémité (54) placée près dudit point de pivot (52) et ayant une deuxième extrémité (56) qui s'étend radialement vers l'extérieur à partir dudit point de pivot (52);
(c) des moyens de guidage courbes (26) ayant une première extrémité (58) fixée rigidement à ladite deuxième extrémité (56) dudit bras pivotant (24) ou venu d'une seule pièce avec elle, lesdits moyens de guidage courbes (26) ayant une deuxième extrémité prolongée (60) qui fait saillie vers l'extérieur en porte-à-faux sur ledit bras pivotant (24), lesdits moyens de guidage courbes (26) se trouvant généralement dans un arc défini par une trajectoire de rotation de ladite deuxième extrémité (56) dudit bras pivotant (24) lorsque ledit bras pivotant (24) est mis en rotation autour dudit point de pivot (52), ledit point de pivot (52) étant placé près d'une saillie ou d'un bord de ladite structure de support (22);
(d) des moyens (28) servant à tailler ladite matière dans les limites dudit perçage courbe ;
(e) des moyens (64) servant à fixer lesdits moyens de taille (28) à ladite deuxième extrémité (60) desdits moyens de guidage courbes (26);
(f) des moyens (30) destinés à commander à distance la rotation dudit bras pivotant (24), et
les moyens de taille (28) comprenant une tête de taille et un support flexible d'énergie de perçage (68) destinés à être reliés à une source d'énergie éloignée, ladite tête de taille étant adaptée pour avancer et se rétracter sur une trajectoire prédéterminée, tout d'abord vers l'extérieur en s'éloignant de la structure de support, puis au moins partiellement vers l'intérieur en se rapprochant de la structure de support, et caractérisé en ce que les moyens de guidage courbes (26) possèdent un conduit (65) dans lequel ledit support flexible d'énergie de perçage (68) desdits moyens de taille (28) est guidé, logé et retenu ledit conduit (65) logeant, retenant et libérant ledit support flexible d'énergie de perçage (68) lorsque lesdits moyens de taille (28) et lesdits moyens de guidage courbes (26) sont avancés et rétractés pour percer un perçage courbe à travers ladite matière,
et en ce que lesdits moyens de commande (30) comprennent une bielle ou une tringlerie de poussée/traction flexible fixée de façon pivotante, soit audit bras pivotant, soit auxdits moyens de guidage courbes, le mouvement de ladite bielle ou tringlerie amenant ledit bras pivotant lesdits moyens de guidage courbes et ladite tête de taille à avancer ou à se rétracter sur ladite trajectoire prédéterminée.

2. Appareil selon la revendication 1, dans lequel ledit support flexible d'énergie de perçage (68) est monté dans ladite structure de support (22) pour décrire un mouvement linéaire dans le sens qui le rapproche et qui l'éloigne desdits moyens de guidage courbes (26).

3. Appareil selon une quelconque des revendications précédentes, dans lequel ladite structure de support (22) contient une unité (36) formant boîtier tenue à la main.

4. Appareil selon une quelconque des revendications précédentes, dans lequel les moyens de taille (28) incorporent un processus d'usinage mécanique, électrochimique ou thermique.

5. Appareil selon une quelconque des revendications précédentes, dans lequel les moyens de commande (30) comprennent au moins un système à manivelle (140 ou 141).

6. Appareil selon une quelconque des revendications précédentes, dans lequel les moyens de commande (30) déplacent entièrement la bielle (134) en translation selon une trajectoire courbe.

7. Appareil selon une quelconque des revendications précédentes, dans lequel les moyens de commande comprennent au moins deux systèmes à manivelle (140, 141), la bielle (134) état articulée aux deux systèmes à manivelle, de façon que chaque point de la bielle circule le long d'une trajectoire circulaire.

8. Appareil selon une quelconque des revendications précédentes, dans lequel les moyens de commande (30) entraînent les moyens de taille (28) en mouvement sur un angle d'environ 180°.

9. Appareil selon une quelconque des revendications précédentes, dans lequel les moyens de commande (30) comprennent en outre un levier ou une gâchette fixée de façon pivotante à la structure de support (22), la bielle (134) étant fixée fonctionnellement audit levier de telle manière que le mouvement dudit levier amène la tige de poussée à se déplacer en translation.

10. Appareil selon une quelconque des revendications précédentes, dans lequel les moyens de commande (30) comprennent en outre une crémaillère et un pignon en prise entre eux, le mouvement longitudinal de ladite crémaillère (192) faisant tourner ledit pignon (188), lequel, à son tour, amène ladite bielle (134) à se déplacer en translation.

11. Appareil selon une quelconque des revendications précédentes, dans lequel la bielle (134) est fixée au bras pivotant (24) ou aux moyens de guidage courbes (26) à la jonction entre le bras pivotant et les moyens de guidage courbes.

12. Appareil selon une quelconque des revendications précédentes, dans lequel les moyens de commande (30) comprennent un mécanisme à manivelles doubles parallèles (140, 141).

13. Appareil selon la revendication 12, dans lequel ledit mécanisme à manivelles doubles parallèles comprend :
(a) ladite structure de support (22);
(b) ledit bras pivotant (24) qui est fixé de façon pivotante à ladite structure de support au droit d'un point de pivot fixe;
(c) un premier système à manivelle (140) fixé de façon rotative à ladite structure de support;
(d) un deuxième système à manivelle (141) fixé de façon rotative à ladite structure de support;
(e) ladite bielle (134) ayant une première extrémité, un point intermédiaire situé sur la longueur de ladite bielle, et une deuxième extrémité, ladite première extrémité de ladite bielle étant fixée de façon pivotante audit bras pivotant (24), ledit premier système à manivelle étant fixé de façon pivotante à ladite bielle audit point intermédiaire, ladite deuxième extrémité de ladite bielle étant reliée de façon pivotante audit deuxième système à manivelle, ladite structure de support, ledit bras pivotant, ladite bielle, ledit premier système à manivelle et ledit deuxième système à manivelle formant un parallélogramme plan ; et
(f) des moyens destinés à faire tourner lesdits premier et deuxième systèmes à manivelle, lesdits moyens de rotation étant fixés au moins au premier système à manivelle.

14. Appareil selon la revendication 13, dans lequel lesdits moyens de rotation comprennent au moins une corde de traction (160, 166) fixée audit premier système à manivelle (140), le mouvement longitudinal de ladite corde de traction faisant tourner ledit premier système à manivelle, lequel, à son tour, amène ladite bielle (134) à faire tourner ledit bras pivotant (24) et ledit deuxième système à manivelle (141).

15. Appareil selon la revendication 14, dans lequel ledit premier système à manivelle (140) comprend au moins une poulie (154), ledit premier système à manivelle et ladite poulie ayant le même axe de rotation, ladite corde de traction (160) étant passée autour de ladite poulie.

16. Appareil selon la revendication 14, comprenant en outre un levier ou une gâchette (174) fixé de façon pivotante à la structure de support (22), le câble de traction (166) état fixé fonctionnellement audit levier de telle manière que le mouvement dudit levier amène la corde de traction à se déplacer longitudinalement et à faire ainsi tourner le premier système à manivelle (140).

17. Appareil selon la revendication 12, comprenant aussi un ressort fixé fonctionnellement à ladite structure de support (22), ledit ressort sollicitant ledit premier ou ledit deuxième système à manivelle (140 ou 141) pour qu'il tourne ou qu'il se rétracte en partant d'une rotation antérieure.

18. Procédé pour former un perçage courbe à l'aide d'un appareil selon une quelconque des revendications précédentes, à l'exclusion de tout procédé relevant de l'article 52(4) EPC.

19. Procédé pour percer un perçage courbe dans une matière, lequel procédé consiste à : déplacer un support flexible d'énergie de perçage allongé (68) le long d'une première direction ou d'une trajectoire d'approche en se rapprochant et en s'éloignant de moyens de guidage courbes (26), lesdits moyens de guidage (26) étant supportés par un moyen pivotant (24) qui se trouve de façon générale dans un arc défini par une trajectoire de rotation centrée sur un axe de rotation ; et commander à distance la rotation desdits moyens de guidage courbes (26) en entraînant en translation une bielle (134) reliée de façon pivotante aux moyens de guidage courbes le long d'une trajectoire courbe d'une amplitude de plus de 90°, et dévier le support flexible d'énergie de perçage (68) par coopération avec les moyens de guidage courbes (26) pour qu'il passe le long d'une trajectoire curviligne ayant une courbure de plus de 90° définie par la trajectoire de rotation centrée autour de l'axe de rotation, en même temps qu'on transmet de l'énergie de perçage le long du support flexible d'énergie de perçage allongé (68) à une tête de taille (61) fixée auxdits moyens de guidage courbes (26) et disposée à une extrémité dudit support d'énergie de perçage (68) et faire ainsi avancer la tête de taille (61) vers la matière pour percer un trou le long de la trajectoire courbe prédéterminée dans la matière, et rétracter la tête de taille (61) jusqu'à une position initiale, en extrayant ainsi les moyens de guidage courbes (26) et la tête de taille (61) de l'intérieur de la matière; mais à l'exclusion de tout procédé relevant de l'article 52(4) EPC.
